(19) Europäisches Patentamt
European Patent Office
Office européen des brevets

(11) **EP 2 121 055 B1**

(12) **EUROPEAN PATENT SPECIFICATION**

(45) Date of publication and mention of the grant of the patent:
**02.04.2014 Bulletin 2014/14**

(51) Int Cl.:
***A61L 31/08*** *(2006.01)*

(21) Application number: **08729527.5**

(22) Date of filing: **11.02.2008**

(86) International application number:
**PCT/US2008/053578**

(87) International publication number:
**WO 2008/100852 (21.08.2008 Gazette 2008/34)**

(54) **MRI COMPATIBLE, RADIOPAQUE ALLOYS FOR USE IN MEDICAL DEVICES**

MRT-KOMPATIBLE UND RADIOPAKE LEGIERUNGEN ZUR VERWENDUNG IN MEDIZINISCHEN VORRICHTUNGEN

ALLIAGES RADIO-OPAQUES COMPATIBLES AVEC L'IRM À UTILISER DANS LES DISPOSITIFS MÉDICAUX

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.02.2007 US 901568 P**

(43) Date of publication of application:
**25.11.2009 Bulletin 2009/48**

(73) Proprietor: **Abbott Cardiovascular Systems Inc.**
**Santa Clara, CA 95054-2807 (US)**

(72) Inventors:
• **PACETTI, Stephen, Dirk**
**San Jose, California 95130 (US)**
• **KRAMER-BROWN, Pamela, Ann**
**San Jose, California 95121 (US)**
• **SANTOS, Ryan, John**
**Corona, CA 92880 (US)**
• **MORRIS, JR., John, William**
**Oakland, California 94618 (US)**
• **MALIK, Shamim, Muhammad**
**Temecula, California 92951 (US)**

(74) Representative: **Boult Wade Tennant**
**Verulam Gardens**
**70 Gray's Inn Road**
**London WC1X 8BT (GB)**

(56) References cited:
| | |
|---|---|
| WO-A1-02/05863 | WO-A1-02/43787 |
| WO-A1-2006/130317 | WO-A2-95/30384 |
| WO-A2-2006/093804 | US-A- 5 477 864 |
| US-A1- 2002 082 681 | US-A1- 2005 070 990 |
| US-A1- 2005 098 241 | US-A1- 2006 153 729 |
| US-A1- 2006 196 581 | US-B1- 6 200 685 |
| US-B1- 6 638 301 | |

**Description**

BACKGROUND OF THE INTENTION

This invention relates to medical devices for use in a human body. In particular, the invention relates to, but is not limited to, medical devices for treating or repairing the body including metal stents that can be viewer effectively by fluoroscopy (x-ray), computer tomography (x-ray based), and magnetic resonance imaging (MRI).

Currently, x-ray (fluoroscopy, computer tomography (CT) and electron beam tomography (EBT)) is the preferred imaging modality for cardiovascular interventional procedures, such as delivering and implanting stents. Stent placement requires real time visualización so that the interventionalist can track the delivery device through the anatomy and place the stent precisely at the target site. Additionally, visualization is necessary after intervention if thrombosis or restenosis occurs. The visualization and tracking of stents under fluoroscopy, which is the most pervasive modality for visualization, is accomplished either through the stent's inherent absorption of x-rays or by the placement of radiopaque markers on the stent. Similarly, fluoroscopy is the preferred imaging modality for viewing anastamosis clips (during follow up) and AAA grafts (during placement and follow up).

At this time, no other method of visualization has the temporal or spatial resolution of fluoroscopy. Nonetheless, fluoroscopy has its drawbacks for both patients and clinicians. For example, catheterization is required in order to directly inject a high concentration of iodinated contrast agent into a patient's coronary arteries or other vessels. Systemic administration of the contrast agent is not practical, as it would require too high of a dose. Furthermore, iodinated contrast agents are nephrotoxic, with a low but measurable incidence of short-term renal failure, and allergic reactivity that also serves as a contraindication for certain patients. Additionally, fluoroscopy uses ionizing x-ray radiation, which also comes with the attendant hazards involved in the administration of radiation. This is an issue for the patient during protracted or repeat interventions, and is also a daily issue for medical personnel, who must monitor their own dosage levels and wear lead shielding during procedures. Finally, fluoroscopy is inherently limited in that it generates a two-dimensional projection image of what are three-dimensional structures. Hence, it requires multiple views for the appraisal of complex vasculature.

Another imaging modality, which has the potential to become valuable in the diagnostic imaging of medical devices, is magnetic resonance imaging (MRI) or a subset of this form of imaging, magnetic resonance angiography (MRA). MRI is presently used for diagnostic applications, but interventional MRI is an active area of research. For devices to be seen under MRI, they must be MRI "compatible." In the context of a diagnostic or interventional procedure, this refers to the ability to accurately image a medical device. Schemes for imaging devices by MRI can be classified as active or passive. In active imaging, an electrical circuit is introduced onto the device, however, this presently is not an easily implemented solution for small, free-standing devices such as anastamosis clips, AAA grafts, or stents. In passive imaging, visibility is a function of the magnetic susceptibility of a metallic medical device. MRI visibility is also a function of device design.

MRI technology presents several distinct advantages over fluoroscopy. For instance, MRI does not use ionizing radiation, and does not require catheterization to image the vasculature. Moreover, MRI contrast agents, which may be necessary for optimal resolution, are much less nephrotoxic than iodinated fluoroscopy agents, and are effective when administered intravenously. An additional advantage of MRI is that it is a tomographic imaging technique that generates a three dimensional data set of the imaged tissue. Consequently, the data set can be manipulated to show different imaging planes and slice thicknesses. This permits high quality transverse, coronal and sagittal images to be obtained directly. Additionally, MRI has greater soft tissue contrast and tissue discrimination than computed tomography (CT) or other x-ray based imaging modalities, such as angiography.

The technique of MRI encompasses the detection of certain atomic nuclei (those possessing magnetic dipole moments) utilizing magnetic fields and radio-frequency (RF) radiation. It is similar in some respects to x-ray computed tomography in providing a cross-sectional display of the body anatomy, only with excellent resolution of soft tissue detail. In its current use, the images constitute a distribution map of hydrogen nuclei, which are protons, and their properties, in organs and tissues. However, unlike x-ray computer tomography, MRI does not use ionizing radiation. The fundamental lack of any known hazard associated with the level of the magnetic and radio-frequency fields that are employed renders it possible to make repeated scans. Additionally, any scan plane can readily be selected, including transverse, coronal, and sagittal sections. MRI is, therefore, a safe, non-invasive technique for medical imaging.

While the benefits of employing MRI technology in connection with medical devices are many, there are some challenges and potential disadvantages associated with its use. Because medical devices typically are constructed using electrically conductive materials, they can suffer from what is known as a Faraday Cage effect when they are imaged by MRI. Generically, a Faraday Cage is a box, cage, or array of electrically conductive material intended to shield its contents from electromagnetic radiation. The effectiveness of a Faraday Cage depends on the wave length of the radiation, the size of the mesh in the cage, the conductivity of the cage material, its thickness, and other variables. Stents , for example, act as Faraday Cages in that they screen the stent lumen from the incident RF pulses of the MRI scanner. This prevents the proton spins of water molecules in the stent lumen from being flipped or excited. Consequently, the desired signal from the stent lumen is reduced by this diminution in excitation. Furthermore, the stent Faraday Cage likely impedes the escape of whatever signal is generated in the lumen. Another important issue in imaging metallic devices by MRI is the effect of the device's magnetic susceptibility. For example, if the magnetic susceptibility of the stent metal is very different from that of the surrounding tissue, the magnetic field in the vicinity of the implant will be perturbed. This alters the resonance condition of protons in the vicinity, thus leading to intravoxel dephasing with an attendant loss of signal. The net result with current metallic stents, most of which are stainless steel, is a signal void in the MRI images. Other metallic stents, such as those made from Nitinol, also have noticeable signal loss in the stent lumen due to a combination of Faraday Cage and magnetic susceptibility effects. Similar results occur with other metallic medical devices with the size of the imaging artifact depending on the size of the device, the particular metal used, the amount of metal in the implant, the device's shape or design, and the scan parameters used by the MRI equipment.

Fluoroscopy and MRI imaging both provide for significant advancements in the technology of medical device visualization. Both technologies have their attendant advantages and disadvantages. There is still a prevalent need for medical devices that can be visualized using fluoroscopy, as fluoroscopy will continue to be the imaging method of choice for such devices for at least the near future. However, MRI is now also being used to non-invasively image many regions of the vasculature. For example, comprehensive cardiac MRI exams have demonstrated clinical utility in the areas of overall cardiac function, myocardial wall motion, and myocardial perfusion, which indicates that MRI may become the standard diagnostic tool for heart disease. Further, MRI exams in other parts of the body, including the vascular system, are becoming more routine. For medical devices that are used in such applications, metals are the preferred material. Unfortunately, most metallic medical devices, particularly those made from stainless steel, obliterate MRI images of the anatomy in their vicinity and obscure the surrounding tissue in the image. By reducing the amount of metal in the medical device, alloying the correct combination of metals, or by changing the structure or pattern of the medical device, the Faraday Cage and magnetic susceptibility effects may be reduced, and these visibility limitations may be addressed.

Therefore, in light of the advances in imaging technologies, a medical device that can be meaningfully imaged by MRI in an optimal manner, while also possessing sufficient radiopacity to be visibly using fluoroscopy methods, would be highly advantageous. Additionally, the alloy used to manufacture such a device would necessarily be biocompatible and sufficiently resistant to corrosion, while also having the requisite mechanical and structural properties suitable for a particular application.

US6638301 relates to coated stents, wherein the radiopaque coating layer can be a titanium and/or gold alloy. Additionally, WO95/30384 and WO2006/093804 disclose radiopaque coatings for stents, suitable for MRI imaging, comprising atoms selected from a list comprising titanium.

The present invention provides an alloy having sufficient inherent radiopacity and MRI compatibility for optimal visibility using both MRI and fluoroscopic imaging technologies, while maintaining mechanical properties and biocompatibility.

SUMMARY OF THE INVENTION

According to the present invention there is provided a medical device for use in a body lumen having the features of claim 1.

The present invention provides a medical device formed from an alloy that possesses sufficient degrees of radiopacity and MRI compatibility to be visible using both x-ray (fluoroscopy, CT and EBT) and MRI imaging technology. Alloy families that provide this dual role are aluminum, titanium, zirconium, niobium, molybdenum, silver, indium, hafnium, tantalum, tungsten, iridium, platinum and gold. These families of elements are selected based on the magnetic susceptibility of the elements. All of the alloys are fluoroscopically compatible, with the elements having a higher atomic mass being more radiopaque under fluoroscopy, while the elements having a lower atomic mass, such as aluminum and titanium, requiring a combination with elements having a greater atomic mass in order to achieve the desired visualization under fluoroscopy.

The specific composition of the alloy of the present invention can vary, depending upon factors that include visibility under fluoroscopy and MRI, biocompatibility, structural characteristics, and the flexibility of a medical device made from the metallic alloy. Accordingly, some ternary and quaternary alloys are suitable alloys of the present invention.

For example, some titanium-based alloys include Ti-29Nb-13Ta-4.6Zr, Ti-35Nb-7Zr-5Ta, Ti-16Nb-13Ta-4Mo, Ti-29Nb-13Ta, Ti-15Nb-6Hf-6Mo, Ti-38Nb-12Al, and Ti-13Zr-13Nb.

As an example, in one embodiment, stents made of pure titanium possess excellent MRI compatibility and biocompatibility, however, they are not sufficiently radiopaque for viewing under fluoroscopy and titanium does not possess the ductility and elongation of stainless steel requiring that the stent pattern be modified to impose less strain. Such modifications require either the stent have a more open pattern or that the stent cannot be expanded to as large a size, both of which compromise its clinical functionality. The alloy composition of the present invention permits the stent to maintain a low delivery profile and to fulfill all of the mechanical and structural requirements attendant to its function as a stent after expansion and deployment in the vessel lumen. However, the alloy composition also provides the stent with an advantage over prior art stent alloys in that it is sufficiently radiopaque and MRI compatible to allow for good imaging of the stent under fluoroscopy and MRI without requiring greater strut thickness or the addition of an extra layer of radiopaque or MRI compatible material or markers. Additionally, the radiopacity under fluoroscopy and the MRI artifact are not so great as to obscure the image of the surrounding anatomy or vessel lumen into which the stent is placed. At the same time, the resulting alloy maintains excellent biocompatibility. In one embodiment, titanium, the largest elemental component is titanium on a volume percentage basis, is alloyed with elements such as tantalum, zirconium, and/or hafnium. It is also contemplated that the added elements may be molybdenum, aluminum, tungsten, iridium, platinum, gold, palladium, and/or silver, or any transition metal from the periodic table of elements with the exception of mercury, cadmium, osmium and copper. The resulting alloy may be binary, wherein titanium is alloyed with a single added element from the above group, or may be composed of any combination, or all, of the above added elements.

The titanium base of the alloy provides it with MRI compatibility for visibility under MRI imaging, and lends the alloy the biocompatibility required for a variety of long term deployment scenarios within a patient's vasculature. However, when these added elements are combined in alloy form with titanium the newly formed alloy also has a greater resistance to corrosion, has better mechanical properties, and changes the radiopacity from that of pure titanium than if pure titanium was used. As a result, the alloy is particularly well suited for use in the formation of stents, anastomosis clips, AAA grafts, and other medical devices to be used in applications in coronary, neurological, saphenous vein graft, renal, iliac, esophageal, biliary, aorta, or other regions of the body.

These and other features and advantages of the invention will become apparent from consideration of the following detailed description which illustrates by way of example the principles of the invention.

DETAILED DESCRIPTION F THE PREFERRED EMBODIMENTS

The present invention relates to a metallic alloy that provides a sufficient degree of radiopacity and MRI compatibility to be visible using both fluoroscopy and MRI imaging technology. The metallic alloy is particularly suitable for medical devices including, but not limited to, intravascular stents, anastomosis clips, and AAA grafts. As an example by comparison, the medical device formed of 316L stainless steel will have some radiopacity under fluoroscopy, however, the same device will generate a "black hole" imaging artifact around the metal device when viewed under MRI imagery. The alloy of the present invention solves the prior art problems by providing a medical device formed of the alloy of the present invention that is compatible with both fluoroscopy and MRI imaging.

Metallic alloy families that provide sufficient radiopacity under both fluoroscopy and MRI imaging include aluminum (Al), titanium (Ti), zirconium (Zr), niobium (Nb), molybdenum (Mo), silver (Ag), indium (In), hafnium (Hf), tantalum (Ta), tungsten (W), iridium (Ir), platinum (Pt), and gold (Au). These alloy families were selected based on the magnetic susceptibility of the elements. All of these alloy families are visible under fluoroscopy, with the metallic elements having a greater atomic mass being more visible and the elements with a lower atomic mass, such as A1 and Ti, requiring a combination with elements having greater atomic mass to provide adequate visualization and radiopacity under fluoroscopy.

In Table 1 immediately below, a list of alloys and elements are compared to 316L stainless steel for comparison of volumetric magnetic susceptibility:

TABLE 1

| Material | Volumetric Magnetic Susceptibility X 10$^{-6}$ |
| --- | --- |
| 316L Stainless Steel | 3500-6700 |
| Elgiloy | 3263 |
| MP35N | 922 |
| Palladium | 813 |
| Platinum | 279 |
| Nitinol | 245 |
| Niobium | 237 |
| Titanium | 182 |
| Tantalum | 178 |
| Molybdenum | 121 |
| Zirconium | 109 |
| Tungsten | 78 |
| Hafnium | 70 |

(continued)

| Material | Volumetric Magnetic Susceptibility X 10$^{-6}$ |
|---|---|
| Iridium | 38 |
| Aluminum | 21 |
| Silver | - 24 |
| Gold | - 34 |
| Indium | - 51 |

As can be seen in Table 1, the volumetric magnetic susceptibility of the metals comprising the alloys of the present invention are substantially lower than those of 316L stainless steel. Accordingly, such alloys when formed into medical devices will be more compatible with MRI imaging.

Binary Alloys

Certain binary alloys of the present invention also demonstrate low magnetic susceptibility while also being visible under fluoroscopy. For example, combining Ti and Ta in any quantity (from pure Ti to pure Ta) covers alloys that are currently used in the aerospace and nuclear power industry as well as in medical devices. Tantalum, for example, is known as an implant material having low magnetic susceptibility. In Table 2 below, certain binary alloys having both good radiopacity for fluoroscopy and compatibility with MRI imaging are compared to annealed 316L stainless steel.

TABLE 2

| Material | Tensile Strength (ksi) | Yield Strength (ksi) | % Elongation |
|---|---|---|---|
| Ti-45Nb | 79 | 70 | 23 |
| Ti-30Ta | 108 | 85 | 26 |
| Ti-40Ta | 118 | 83 | 23 |
| Ti-50Ta | 100 | 86 | 24 |
| Ti-60Ta | 93 | 61 | 24 |
| Annealed 316L | 85 | 40 | 50 |

While the tensile strength, yield strength and percent elongation of the binary alloys listed in Table 2 compare favorably with that of annealed 316L stainless steel, the radiopacity under fluoroscopy and compatibility with MRI imaging is substantially better than that of the 316L stainless steel. Medical devices made from these binary alloys should demonstrate low magnetic susceptibility in addition to having sufficient radiopacity to be visible under fluoroscopy.

Ternary and Quaternary Alloys

Certain ternary and quaternary alloys also demonstrate low magnetic susceptibility for MRI imaging and provide sufficient radiopacity for fluoroscopic imaging as well. For example, some Ti-based alloys that cam be used to form medical devices include: Ti-29Nb-13Ta-4.6Zr, Ti-35Nb-7Zr-5Ta, Ti-16Nb-13Ta-4Mo, Ti-29Nb-13Ta, Ti-15Nb-6Hf-6Mo, Ti-38Nb-12Al, Ti-13Zr-13Nb, Ti-9Mo-9Hf, Ti-7Mo-7Nb-7Hf, and Ti-15Zr-5Nb-4Ta-0.2Pd. The mechanical properties of some of these ternary and quaternary alloys are shown in Table 3 below in comparison to annealed 316L stainless steel.

TABLE 3

| Material | Tensile Strength (ksi) | Yield Strength (ksi) | % Elongation |
|---|---|---|---|
| Ti-29Nb-13Ta-4.6 Zr | 76 | 36 | 40 |
| Ti-35Nb-7Zr-5Ta | 80 | 70 | ≥15 |
| Ti-16Nb-13Ta-4Mo | 94 | 80 | 65 |
| Ti-29Nb-13Ta | 82 | 29 | 32 |
| Ti-15Nb-6Hf 6Mo | 105 | 83 | 46 |
| Ti-12Al-38Nb | 82 | 80 | >27 |
| Ti-13Zr-13Nb | 103 | 69 | 26 |
| Ti-9Mo-9Hf | 119 | 106 | 44 |
| Ti-7Mo-7Nb-7Hf | 114 | 90 | 41 |

(continued)

| Material | Tensile Strength (ksi) | Yield Strength (ksi) | % Elongation |
|---|---|---|---|
| Ti-15Zr-5Nb-4Ta-0.2Pd | 104 | 101 | 28 |
| Annealed 316L | 85 | 40 | 50 |

The alloy of the present invention is particularly useful for use in intravascular stents. At present, there are numerous stents that are commercially available that would benefit from the alloy of the present invention. As examples, the alloy of the present invention could be used to make commercially available stents having the tradenames MultiLink (family of stents manufactured by Advanced Cardiovascular Systems, Inc., Santa Clara, California), NIR and Express (marketed by Boston Scientific Corporation, Natick, Massachusetts), BX Velocity (marketed by Cordis Corp., Miami Lakes, Florida), S670 and S7 (marketed by AVE/Medtronic, Santa Rosa, California). The alloy of the present invention also would benefit the stent patterns disclosed in, for example, U.S. Patent Nos. 4,733,665 (Palmaz); 4,739,762 (Palmaz); 5,102,417 (Palmaz); 5,292,331 (Boneau); 5,653,727 (Wiktor); 5,195,984 (Schatz); 4,580,568 (Gianturco); 5,421,955 (Lau); 5,514,154 (Lau); 5,733,303 (Israel); and 6,190,403 (Fischell) .

The Stent Example

In one exemplary alloy of the invention, as applied to stents, the stent incorporates properties of certain titanium alloys that are both compatible with MRI imaging and sufficiently radiopaque for imaging by x-ray (fluoroscopy, CT and EBT). Stents produced from these alloys are constructed using specifically tailored alloy compositions which are beneficial in that the stents are sufficiently radiopaque and MRI compatible to be visualized, yet the stent's radiopacity and MRI artifact are not so great as to interfere with the visualization of the surrounding body tissue or lumen.

The stent can have virtually any configuration that is compatible with the vessel lumen in which it is implanted. Typically stents are composed of an intricate geometric pattern of cylindrical rings and connecting links. These elements are commonly referred to as struts. Generally, the struts are arranged in patterns which are designed to contact the lumen walls of a vessel and to maintain patency of the vessel thereby. A myriad of strut patterns are known in the art (see above) for achieving particular design goals. A few of the more important design characteristics of stents are radial or hoop strength, expansion ratio or coverage area, and longitudinal flexibility. One strut pattern may be selected over another in an effort to optimize parameters that are of importance for a particular application.

The current stent market mainly consists of stents made from stainless steel, nickel-titanium, and tantalum, and generally may be either self-expanding or expandable by some external means such as a balloon that is affixed to a balloon catheter. Stents generally must be capable of expansion because they are optimally delivered to the stenotic region through the tortuous vasculature of the patient after percutaneous access has been achieved. It is advantageous that the stent be crimped onto a catheter so that it is maintained in a contracted state, or low profile, during delivery through the vasculature. This affords the physician greater ease of direct placement without pre-dilatation, and minimizes trauma to the vessel wall and disruption in blood flow throughout delivery and deployment. However, for a physician to precisely place the stent at the deployment site, deliver it through the vasculature with minimal trauma to the vessels through which it passes, and to monitor and diagnose the patient after a placement, he or she must be able to adequately visualize the stent within the patient's body.

The present invention provides the physician with a visually versatile stent that can be imaged by different technologies depending on the application at hand. For example, a physician may rely on the stent's radiopacity when using fluoroscopy to visualize the stent during delivery and placement, while later relying on the stent's MRI compatibility for visualization by MRI during subsequent diagnostic and monitoring visits with the patient, thereby taking advantage of the non-invasiveness of MRI. This versatility is especially beneficial in that MRI use is increasing, with the comprehensive cardiac MRI exam having the potential to become a standard diagnostic tool. As a result, placement of a stent under fluoroscopy with subsequent diagnostic imaging being accomplished by MRI is a distinctly possible medical protocol that may emerge in the foreseeable future. It is also possible that cardiac interventions under MRI imaging will develop.

Titanium alloys, especially nickel-titanium or nitinol, are well known for use in the construction of self-expanding stents. These alloys provide serviceable radiopacity and fairly good MRI compatibility. Titanium also possesses some favorable qualities for use in constructing balloon expandable stents. For instance, pure titanium is MRI compatible and is regarded as one of the most biocompatible metals.

However, titanium also has some disadvantages associated with its use in balloon expandible stent applications. Titanium has a lower tensile strength and elongation than, for example, stainless steel. More importantly, titanium is a low density element and is, therefore, not sufficiently radiopaque for viewing by fluoroscopy. This deficiency can be overcome by increasing the strut thickness, as greater strut thickness imparts greater radiopacity to the stent. For example, testing has revealed that good radiopacity for a stainless steel stent is attained with a strut thickness of approximately 0.0058 inch (0.1473 mm), while good radiopacity for a titanium stent is attained at 0.0159 inch (0.4039 mm). In other words, stainless steel is nearly three times more radiopaque than titanium. However, although it is possible to increase titanium's radiopacity by increasing the strut thickness of the stent, this is not desirable because it significantly increases stent profile and may decrease stent flexibility. Therefore, it is desirable to alloy titanium with an appreciable level of another element, generally a higher density transition metal, that will confer enough radiopacity to the alloy to produce a sufficiently radiopaque stent.

In one embodiment, elements alloyed with titanium are tantalum, zirconium, hafnium and niobium. Additionally, it is also contemplated that the advantages of the present invention may be attained by alloying titanium with molybdenum, aluminum, tungsten, iridium, platinum, gold, palladium, and silver. The combination of metals used in making the alloy may be binary, wherein titanium is combined with a single added element, or may be a combination of titanium with any number, or all, of the added elements. In each case, the aim of producing such an alloy is to improve the radiopacity and mechanical properties over those of the original element, while preserving biocompatibility and MRI compatibility. It is also desirable that the resulting alloy provide enhanced resistance to corrosion. Accordingly, the advantages provided by the present invention may be discussed in terms of these attributes.

Pure titanium and tantalum are well known for use in permanently implanted medical devices due to their excellent biocompatibility and corrosion resistance. Titanium, tantalum, niobium, zirconium, and hafnium, are all very electropositive elements, which form an extremely stable oxide film on their surfaces. As a result, a stent composed of an alloy containing these elements, such as the present invention, maintains excellent resistance to corrosion, inertness, and biocompatibility. Titanium, niobium, and tantalum have been extensively studied for orthopedic applications due to these qualities. Even elements that are not normally biocompatible by themselves, such as aluminum and molybdenum, are adequately sequestered when alloyed with titanium, with so little corrosion taking place that no known negative biological effect has been associated with release of these elements. This is evidenced by the fact that the most common alloy used in constructing hip implants is TIVANIUM®, Ti-6Al-4V.

The alloy of the present invention will be similarly resistant to corrosion. Zirconium has excellent corrosion resistance to HCl, NaOH, $HNO_3$ and pure $H_2SO_4$- More particularly, zirconium resists boiling HCl, even though HCl solution dissolves 316L stainless steel rapidly. Additionally, in boiling concentrated HCl, tantalum displays no measurable corrosion, indicating that it is quite capable of handling highly corrosive media. Niobium's corrosion properties resemble those of tantalum, but are not quite as good at elevated temperatures well above body temperature. Hafnium, though lesser used due to its scarcity, is used as control rods for nuclear reactors, which is self-evident of its corrosive resistance under extreme conditions. Therefore, alloys constructed from titanium and the added elements of the present invention, especially after surface electropolishing, are expected to be highly resistant to corrosion.

Of particular importance to the present invention, the titanium based alloy will produce a stent that is MRI compatible. The "compatibility" of a stent in MRI imaging refers to the ability to visualize the stent with adequate contrast and resolution. The present invention alloy stent will be viewed in an MRI image by the formation of imaging artifacts, the sources of which include magnetic susceptibility and electrical conductivity or the Faraday Cage effect. Any metal that has a magnetic susceptibility different from that of the surrounding tissue will generate a susceptibility artifact. The magnitude of the artifact depends on how much the susceptibility differs from that of tissue. These artifacts usually are signal voids or dark spots on the image. Electrically conductive metals in an MRI scanner can also have electrical currents induced in them by the radio frequency pulses. For stents, this can lead to the stent shielding the lumen from the radio frequency excitation signal. Stents made of the present invention composition are amongst the most MRI compatible, while the prior art stainless steel stents are the least MRI compatible.

A larger difference in magnetic susceptibility between the stent and the surrounding tissue leads to a larger artifact. A large artifact makes the stent easy to locate, but may extend beyond the stent, covering the entire vessel and obscuring the lumen, making precise determination of the stent location or degree of expansion difficult. A small artifact enables accurate assessment of the stent dimensions and lumen. In this case, the stent itself is somewhat harder to locate initially, but when found, the tissue in proximity can be visualized well. It must be noted that a stent made in accordance with the present invention will be impacted by the concerns inherently presented by the Faraday cage effect, which is created by the electrical conductivity of metallic stents and prevents RF signals from penetrating the stent lumen in MRI imaging. However, these concerns are present in any metallic stent application, and can be overcome by correct selection of MRI pulse sequence and RF pulse power.

The closer the stent magnetic susceptibility is to that of blood, the smaller the artifact, which produces an MRI image of optimal contrast and resolution. When compared with stents made from Nitinol or 316L stainless steel, the most common alloys for stents, titanium and the four added elements of this embodiment, tantalum, niobium, zirconium, and hafnium, all have individual magnetic susceptibilities that are closer to blood. Also, it is known that titanium is MRI compatible. Accordingly, it follows that the titanium alloys of the present invention provide a stent having an MRI compatibility comparable to one made of pure titanium.

Finally, alloying the added elements of this embodiment with titanium affords the stent manufacturer a radiopacity that is not present in pure titanium. In the design of stents, there is a highly positive correlation between material density and radiopacity. Thus, the greater a material's density, the greater the material's radiopacity under fluoroscopy. Titanium, due to its low density, acts as a very effective diluent, lowering the density of any resulting alloy in which it is the base element. This effect is desirable in, for instance, aerospace applications, but is not desirable in stent applications, where the visibility of such a small medical device inside the vasculature of a patient is imperative, and requires that the stent be sufficiently radiopaque to be visible under fluoroscopy. In terms of stent applications, therefore, to be practical, the material used in manufacturing the stent must be at least as radiopaque as 316L stainless steel. If it is not, the material's density must be increased by alloying it with other elements.

In the alternative, if a stent material is not sufficiently radiopaque, additional radiopacity may be gained by increasing the thickness or width of the struts of the stent itself, or in other words, by simply using more metal in the stent. There is a generally linear relationship between material thickness and radiopacity. Thus, for two stents of the same stent pattern and material, but having different strut thicknesses, the stent with the greater strut thickness will be proportionately more radiopaque. However, increasing the size of the struts is undesirable as a stent's profile is compromised in terms of flexibility and deliverability. The larger a stent's profile, the more difficult it is to navigate it through the vasculature during delivery and placement. Therefore, in stent applications, it is more desirable to increase a material's radiopacity by using a radiopaque alloy in constructing the stent, while only minimally increasing the resulting strut dimensions, if at all.

It is also possible for a stent to be too radiopaque and, therefore, undesirable because the lumen of the stent cannot be adequately imaged by fluoroscopy. More specifically, after implantation, the lumen cannot be imaged by passage of contrast agent through the stent, making determination of in-stent restenosis or thrombosis very difficult using current fluoroscopy techniques. As a result, the elements added to the titanium based alloy of this embodiment are selected to increase the density of the resulting stent struts, ensuring that the alloy is within a range of radiopacity that is sufficiently radiopaque to permit visibility under fluoroscopy, but not so radiopaque as to obscure the stent lumen and surrounding tissue from visibility.

For the purposes of the present invention, a desirable or useful alloy radiopacity range has been calculated. This range permits a manufacturer to adjust the density or strut thickness of the resulting stent based on the application at hand. A stent made from 316L stainless steel, having a strut thickness of about 0.0022 inch (0.0559 mm) has been determined to represent the approximate lower limit of radiopacity, below which the stent is insufficiently radiopaque to be observed. Likewise, 316L stainless steel stent struts having a thickness of about 0.009 inch (0.2286 mm) represent the upper limit of radiopacity, above which the stent is too bright to enable visualization of the lumen. Acceptable radiopacity of 316L stainless steel is achieved in a stent with strut thicknesses of approximately 0.0058 inch (0.1473 mm).

The radial or hoop strength of a stent varies, generally, as a function of the strut thickness cubed ($t^3$). For this reason, stents utilizing identical stent patterns and being designed to have substantially the same radial strength may have comparatively large differences in strut thickness as result of apparently small differences in material yield strength. While the mechanical strength of the alloy determines the practical minimum strut thickness, a reasonable lower boundary is about 0.002 inch (0.0508 mm) thick based on 316L stainless steel. Additionally, for both coronary and peripheral applications, a reasonable upper limit on strut thickness is about 0.009 inch (0.2286 mm). With this in mind, a useful radiopacity window can be defined for alloys based on strut thickness. This range is summarized in Chart 1, which illustrates how the range of useful alloy radiopacity is affected by strut thickness:

## Chart 1:

**Range of Useful Alloy Radiopacity**

Strut Thickness (mils)

▨ Too Radiopaque
■ Good Radiopacity
◩ Insufficient Radiopacity

A definition of the alloy radiopacity coordinate of Chart 1 requires the use of a calculation model for determinine radiopacity. Such a model is possible based on a corrected mass absorption coefficient, as radiopacity is related to the amount of x-ray energy absorbed by the inner shell electrons of a particular element All fluoroscopic imaging systems used in interventional cardiology use an x-ray tube with a tungsten source. Therefore, a characteristic tungsten x-ray spectrum is employed to determine a corrected mass absorption coefficient for each element. These corrected values are related to each alloyed element's relative radiopacity. In order to determine alloy relative radiopacity, the volume percentages of the alloy elements are used in combination with the elemental relative radiopacity. Since the x-ray absorption rates of the elements in an alloy are independent of one another, their individual contributions can be simply added. Also, since the absorption of x-rays increases exponentially with linearly increasing thickness, the relative radiopacity value is combined with the thickness, of the material to determine a value for the percentage of x-ray energy absorbed.

A range for the maximum and minimum acceptable x-ray absorption for stents contemplated by the present invention has been determined, based on the above calculational model, experimentation, studies, and observations, to be between the limits of 1.4% and 5.6 %. Therefore, to calculate, for example, if the total x-ray absorption for an alloy incorporating the preferred added elements lies within the acceptable range for the present invention, the following calculation is used:

$$0.014 \ddot{y} 1 - e^{-[\text{Strut Thickness (cm)} \times \{ (Ti)(C_1) + (Zr)(C_2) + (Nb)(C_3) + (Hf)(C_4) + (Ta)(C_5) \}]} \leq 0.056$$

This equation states that the total x-ray absorption for the radiopaque titanium alloy should be in the range of 1.4% to 5.6%, at a strut thickness of between 0.002 and 0.009 inch (0.0508 and 0.2286 mm).

The terms in the equation (Ti), (Zr), (Nb), (Hf), (Ta), etc., are the volume fraction of the respective elements (volume percent / 100) in the alloy. The volume fraction is used because radiopacity is affected by x-ray path length. The terms in the equation C1, C2, C3, C4, C5, etc., are the x-ray absorption coefficients for the respective elements. The above equation should not be considered exclusive to the elements shown. The titanium based alloys of the present invention contemplate that this equation may be modified to include or exclude any of the added elements in the preferred set of tantalum, niobium, zirconium, and hafnium, and/or the additionally contemplated elements of molybdenum, aluminum, tungsten, iridium, platinum, gold, palladium, and silver. It is also contemplated that the alloys of the present invention and the above model include all other elements in the second and third row transition metals of the periodic table of elements, such as yttrium, technetium, ruthenium, rhodium, and rhenium. The combination of metals used in making the alloy may be binary, wherein titanium is combined with a single added element, or titanium is combined with any number, or all, of the above mentioned elements.

The above description of the relationships between material density, strut thickness, and radiopacity, are greatly simplified in nature. However, the relationships as described are sufficient to illustrate the advantages of alloying the described added elements with titanium for use in balloon expandable stents, especially for use in increasing the radiopacity of the resulting stent and maintaining its MRI compatibility.

For comparison purposes, an annealed 316L stainless steel stent may be used as a baseline. Such stents have been demonstrated to have sufficient radial strength to perform adequately as a stent over a wide range of initial delivery and expanded diameters, and with a variety of common stent patterns. While stainless steel stents are visible under x-ray fluoroscopy, the degree of visibility varies greatly depending on such factors as the strut dimensions and stent pattern. However, it has been shown, during testing evaluated and performed for the present invention, that the alloying of the above listed added elements with titanium creates a stent with mechanical properties (such as tensile and yield strength) comparable to that of 316L stainless steel, yet also sufficiently radiopaque and MRI compatible for imaging under fluoroscopy and MRI.

For example, in one binary alloy of titanium and tantalum, it has been shown that the incorporation of anywhere from 22% by weight (Ti-22Ta) to 80% by weight (Ti-80Ta) tantalum, with a strut thickness between 0.002 and 0.009 inch (0.0508 and 0.2286 mm), will yield an acceptable radiopacity and MRI compatibility required to visualize the stent in addition to adequate mechanical properties in terms of strength and flexibility. As the percentage of tantalum increases, the strut thickness decreases to maintain ideal radiopacity. Such Ti-Ta alloys are known in the art and have been used in the aerospace and nuclear power industries. In another representative example of a preferred binary alloy of the present invention, titanium is combined with niobium. Such alloys are known and used in super conducting wire and aerospace rivets. A comparison of the mechanical properties of one such alloy with 316L stainless steel is summarized in Table 4:

TABLE 4

| Material | Tensile Strength (ksi) | Yield Strength (ksi) | Elongation |
|---|---|---|---|
| Ti-45Nb | 79 | 70 | 23 |
| Annealed 316L | 85 | 40 | 50 |

As indicated in Table 4, a significant advantage of the present invention is found in the enhanced radiopacity and MRI compatibility it provides without appreciable loss of yield or tensile strength, or a significant increase in strut dimensions. It has been determined that the ideal radiopacity for stents with the practical range of strut thicknesses for Ti-Nb alloys is anywhere from pure Nb to 23% Nb by weight (Ti-23Nb).

It is also contemplated that the present invention may embody any combination of titanium and the specified added elements, and those that do not represent binary alloys. For example, tertiary, quaternary, etc., alloys may be produced with equally effective results, as highlighted in Table 5:

TABLE 5

| Material | Tensile Strength (ksi) | Yield Strength (ksi) | % Elongation |
|---|---|---|---|
| Annealed 316L | 85 | 40 | 50 |
| Ti-29Nb-13Ta | 81.4 | 29 | 30 |
| Ti-12Al-38Nb | 82 | 80 | >27 |
| Ti-13Zr-13Nb | 103 | 69 | 26 |
| Ti-29Nb-13Ta-4.6Zr | 79 | 36 | 40 |
| Ti-35Nb-7Zr-5Ta | 80 | 70 | 15 |
| Ti-16Nb-13Ta-4Mo | 94 | 80 | 65 |
| Ti-15Nb-6Hf-6Mo | 105 | 83 | 46 |

A stent made in accordance with the titanium alloy of the present invention represents a significant advancement in the art of stents, offering significant advantages to the interventionalist, the physician, the manufacturer, and the patient in whom such a stent is placed. The alloy is biocompatible and resistant to corrosion, and is visible both under MRI imaging and fluoroscopy.

In combining suitable percentages of the listed added elements with suitable percentages of titanium to achieve these advantages, the level of radiopacity of the stent can be fine tuned in one of two ways. First, the radiopacity of the stent will be a function of its geometry. The thickness of the stent struts may be adjusted to obtain optimal radiopacity, as may the geometric design of the stent. Second, if the alloy's radiopacity is not adequate, titanium may be alloyed with a higher density metal to obtain such optimal radiopacity.

In one arrangement the titanium and added element content of the alloy will be uniformly distributed continuously in forming and manufacturing the stent. However, it is contemplated that the alloy of the present invention will be non-uniformly distributed throughout the body of a stent constructed using another material to facilitate the desired visibility requirements for the stent application at hand. For instance, the present invention alloy may be concentrated in evenly dispersed annular bands circumscribing the stent, or in bands extending longitudinally along the body of the stent. Finally, it is also contemplated that the alloy of the present invention may also be used as a coating that covers a stent formed from another material. This would confer visibility under fluoroscopy and under MRI if the underlying material is MRI compatible.

The AAA Device Example

Patient's suffering from aortic abdominal aneurysms (AAA) are increasingly being treated with AAA grafts. Typically, such AAA grafts have a tubular portion made from a material such as Dacron™ (polyethylene terephthalate) or Gortex® (made by W.L. Gore) and metallic fasteners that help to attach the tubular portion to the aorta. There are numerous AAA graft designs which are discussed in, for example, U.S. Patent Nos. 6,221,102 (Baker); 6,214,038 (Piplani); 6,210,434 (Quiachon); 6,203,568 (Lombardi); 6,273,909 (Kugler); 6,187,036 (Shaolian); 6,168,610 (Marin); 6,102,940 (Robichon); 6,162,246 (Barone); 5,662,700 (Lazarus).

Minimally invasive procedures to implant AAA grafts are saving lives and dramatically reducing recovery times for patients versus invasive surgery graft options. Patients who have grafts are followed up with on a regular basis using non-invasive procedures to track aneurysm progress. These patients may undergo fluoroscopic or CT (x-ray based) procedures, ultrasound procedures and/or MRI procedures. If a patient with an Elgiloy attachment system undergoes a fluoroscopic procedure, the system shows up visibly under the x-ray, with added visibility and twist indication given by Pt markers that run along the graft length. If the same patient undergoes an MRI procedure, the current Elgiloy components will yield "black hole" or signal loss artifacts around these components while the Pt markers will generate much smaller artifacts. In addition, aspects of the delivery system currently manufactured from less-friendly MRI alloys, such as stainless steel, would also exhibit -ray imaging would allow both interventions and follow-up with different imaging modalities to take place without sacrificing the information that may be obtained from those procedures. This material could be used for the delivery system, the graft attachment system (frame and/or hooks), a graft support system (internal stent or sewn-in stent) and/or the graft twist markers.

As described above, the alloy of the present invention is particularly suitable for use with stents, and it is equally suitable for use with AAA grafts. Thus, the disclosure set forth for the stent example, applies equally to those portions of the AAA graft made from a metallic alloy (attachment system, frame, hooks, and markers).

The Anastomosis Clip Example

Use of anastomosis devices, specifically clips, are gaining in popularity as a surgical device for use in heart bypass operations. Typical vein grafts from such operations last approximately ten years. Increasingly, patients with bypass grafts are undergoing diagnostic procedures for angina and other cardiac related issues. These patients may undergo fluoroscopic procedures and/or they may undergo MRI procedures. If a patient with a 316L stainless steel clip undergoes a fluoroscopic procedure, the clip will show up visibly under the x-ray. If the same patient undergoes an MRI procedure, the clip will generate a "black hole" imaging artifact around the metal contained in the implant. Utilizing a biocompatible, MRI compatible material that also has adequate radiopacity under fluoroscopy would allow follow-up under differing procedures to take place without sacrificing the information that may be obtained from those procedures.

Some examples of anastomosis clips can be found in, by way of example, 6,206,913 (Yencho); 6,113,612 (Swanson); U.S. Publication No. 2002/0022853A1 (Swanson); U.S. Publication No. 2002/0013591A1 (Fleischman); U.S. Publication No. 2001/0047180 A1 (Grundem).

While only the presently preferred embodiments have been described in detail, as will be apparent to those skilled in the art, modifications and improvements may be made to the alloy disclosed herein without departing from the scope of the invention. Accordingly, it is not intended that the invention be limited, except as by the appended claims.

**Claims**

1. A medical device for use in a body lumen, comprising:

> a pattern of struts interconnected to form a structure, at least a portion of the struts contact the walls of the body lumen, the struts having a first composition that is MRI compatible, wherein the struts are non-uniformly coated with an alloy having a second composition different from the first composition, the alloy comprising a relatively higher volume fraction of titanium and a relatively lower volume fraction of one or more added elements, wherein the coating imparts both radiopacity and magnetic resonance imaging compatibility to the device and wherein said non-uniformity results in the alloy being concentrated in evenly dispersed annular bands circumscribing the device or in bands extending longitudinally along the device.

2. The medical device of claim 1, wherein the alloy is binary, comprising titanium and a percentage of one added element selected from a group consisting of tantalum, niobium, zirconium, hafnium, molybdenum, aluminium, tungsten, iridium, platinum, gold, palladium, silver, yttrium, technetium, ruthenium, rhodium and rhenium.

3. The medical device of claim 1 wherein the added element or elements is or are selected from the group consisting of tantalum, niobium, zirconium, hafnium, molybdenum, aluminium, tungsten, iridium, platinum, gold, palladium, silver, yttrium, technetium, ruthenium, rhodium and rhenium or from a group consisting of the transition elements with the exception of mercury, cadmium, osmium and copper.

**Patentansprüche**

1. Medizinprodukt zur Verwendung in einem Körperlumen, das umfasst:

eine Anordnung von Streben, die zur Bildung einer Struktur miteinander verbunden sind, wobei zumindest ein Teil der Streben mit den Wänden des Körperlumens in Kontakt ist, wobei die Streben eine erste Zusammensetzung aufweisen, die mit MRT kompatibel ist, wobei die Streben nicht gleichförmig mit einer Legierung einer von der ersten Zusammensetzung verschiedenen zweiten Zusammensetzung beschichtet sind, wobei die Legierung einen relativ höheren Volumenanteil an Titan und einen relativ niedrigeren Volumenanteil eines oder mehrerer weiterer Elemente aufweist, wobei die Beschichtung sowohl eine Röntgenstrahlenundurchlässigkeit verleiht als auch zur Kompatibilität des Produktes mit der Magnetresonanztomographie führt und wobei die Ungleichförmigkeit dazu führt, dass die Legierung in gleichmäßig verteilten ringförmigen Bändern, die das Produkt umgeben, oder in Bändern, die sich longitudinal entlang des Produktes erstrecken, konzentriert ist.

2. Medizinprodukt nach Anspruch 1, wobei die Legierung eine binäre Legierung ist, die Titan und einen prozentualen Anteil eines weiteren Elements umfasst, das unter Tantal, Niob, Zirconium, Hafnium, Molybdän, Aluminium, Wolfram, Iridium, Platin, Gold, Palladium, Silber, Yttrium, Technetium, Ruthenium, Rhodium und Rhenium ausgewählt ist.

3. Medizinprodukt nach Anspruch 1, wobei das oder die weiteren Elemente unter Tantal, Niob, Zirconium, Hafnium, Molybdän, Aluminium, Wolfram, Iridium, Platin, Gold, Palladium, Silber, Yttrium, Technetium, Ruthenium, Rhodium und Rhenium oder unter den Übergangselementen außer Quecksilber, Cadmium, Osmium und Kupfer ausgewählt ist (sind).

**Revendications**

1. Dispositif médical pour l'utilisation dans une lumière corporelle, comprenant :

un motif d'entretoises interconnectées pour former une structure, au moins une portion des entretoises vient en contact avec les parois de la lumière corporelle, les entretoises ayant une première composition qui est compatible MRI, où les entretoises sont revêtues de manière non uniforme d'un alliage d'une deuxième composition différente de la première composition, l'alliage comprenant une fraction volumique relativement plus élevée de titane et une fraction volumique relativement plus basse d'un ou de plusieurs éléments ajoutés, où le revêtement confère à la fois de la radio-opacité et une comptabilité d'imagerie de résonnance magnétique au dispositif, et où ladite non uniformité se traduit en ce que l'alliage est concentré en bandes annulaires uniformément dispersées circonscrivant le dispositif ou en bandes s'étendant longitudinalement le long du dispositif.

2. Dispositif médical selon la revendication 1, dans lequel l'alliage est binaire, comprenant le titane et un pourcentage d'un élément ajouté sélectionné dans un groupe consistant en tantale, niobium, zirconium, hafnium, molybdène, aluminium, tungstène, iridium, platine, or, palladium, argent, yttrium, technétium, ruthénium, rhodium et rhénium.

3. Dispositif médical selon la revendication 1, où le ou les éléments ajoutés est ou sont sélectionné dans le groupe consistant en tantale, niobium, zirconium, hafnium, molybdène, aluminium, tungstène, iridium, platine, or, palladium, argent, yttrium ,technétium, ruthénium, rhodium et rhénium ou d'un groupe consistant en éléments de transition à l'exception du mercure, cadmium, osmium et cuivre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6638301 B
- WO 9530384 A
- WO 2006093804 A
- US 4733665 A, Palmaz
- US 4739762 A, Palmaz
- US 5102417 A, Palmaz
- US 5292331 A, Boneau
- US 5653727 A, Wiktor
- US 5195984 A, Schatz
- US 4580568 A, Gianturco
- US 5421955 A, Lau
- US 5514154 A, Lau
- US 5733303 A, Israel
- US 6190403 A, Fischell
- US 6221102 B, Baker
- US 6214038 B, Piplani
- US 6210434 B, Quiachon
- US 6203568 B, Lombardi
- US 6273909 B, Kugler
- US 6187036 B, Shaolian
- US 6168610 B, Marin
- US 6102940 B, Robichon
- US 6162246 B, Barone
- US 5662700 B, Lazarus
- US 6206913 B, Yencho
- US 6113612 A, Swanson
- US 20020022853 A1, Swanson)
- US 20020013591 A1, Fleischman
- US 20010047180 A1, Grundem